# EUROPÄISCHE PATENTSCHRIFT

(11) **EP 4 268 493 B1**
(45) Veröffentlichungstag und Bekanntmachung des Hinweises auf die Patenterteilung: **14.10.2026**
(21) Anmeldenummer: 21830977.1
(22) Anmeldetag: 03.12.2021
(51) Int. Cl.: H04W 12/50, H04W 4/80, A61B 5/02, A61B 5/00

(54) **ÜBERTRAGUNGSEINHEIT FÜR BLUETOOTH-PAARUNG**
TRANSMISSION UNIT FOR BLUETOOTH PAIRING
UNITÉ DE TRANSMISSION POUR L'APPAIRAGE BLUETOOTH

(30) Priorität: 24.12.2020 DE 102020216572
(43) Veröffentlichungstag der Anmeldung: 01.11.2023
(73) Patentinhaber: Cosinuss GmbH, 81379 München (DE)
(72) Erfinder: KREUZER, Johannes, 81377 München (DE)
(74) Vertreter: Franke, Dirk
(86) Internationale Anmeldenummer: PCT/EP2021/084282
(87) Internationale Veröffentlichungsnummer: WO 2022/135881

(56) Entgegenhaltungen:
- DE-A1- 102019 001 483
- US-A1- 2015 099 466
- US-A1- 2020 138 288

## Beschreibung

Die vorliegende Erfindung betrifft eine Übertragungseinheit für ein Signal außerhalb der Bandbreite im Rahmen einer Bluetooth-Paarung von mindestens zwei bluetoothfähigen Geräten, ein lichtundurchlässiges Gerät zur Messung von Vitalparametern, ein System und ein Verfahren zur Bluetooth-Paarung.

### Stand der Technik

Die fortschreitende Miniaturisierung erlaubt die Messung von Vitalparametern auch während körperlicher Bewegung, idealerweise über Sensoren im Gehörgang. Diese haben den Vorteil, dass sie im Gegensatz zu Handgelenksensoren stabile und kontinuierliche Messwerte liefern. Handgelenkssensoren hingegen können insbesondere beim Sport starken, die Messwerte verfälschenden Störeinflüssen unterliegen. Vorteilhafterweise lassen sich die Gehörgangsensoren über Bluetooth mit weiteren, bluetoothfähigen Geräten, insbesondere mit Mobiltelefonen, über eine Bluetooth-Schnittstelle drahtlos verbinden.

Kontinuierliche Messungen von Vitalparametern im äußeren Gehörgang werden durchgeführt im Bereich des Individualsports, finden aber zunehmend auch Anwendung im medizinischen Sektor. Das dafür eingesetzte Gerät umfasst einen Lichtemitter, zumeist in Form einer im grünen, roten oder infraroten Bereich emittierenden LED, wobei der Lichtemitter im äußeren Gehörgang an der Haut des Gehörgangs mithilfe eines elastischen Silikonschirmchens platziert wird. An einer davon entfernten Stelle wird das aus dem Gewebe austretende Licht mit einer Photodiode erfasst, in Strom umgewandelt, welcher in einer hinter der Ohrmuschel angeordneten Messeinheit erfasst wird. Diese Messeinheit beherbergt Messtechnik, Stromversorgung des Geräts und einen Prozessor. Analog erfasste Signale werden digitalisiert, verarbeitet und via Bluetooth, ANT+ oder andere Funkprotokolle an entsprechende Empfängergeräte gesendet.

Für die Übertragung von Daten via Bluetooth, insbesondere für die Übertragung von individuell gemessenen und zuzuordnenden Vitalparametern ergibt sich das Problem, dass einerseits aufgrund der Art der zu übertragenden Information im medizinischen Bereich und aufgrund des Datenschutzes eine sichere Datenübertragung und Speicherung zwingend erforderlich ist, andererseits aber auch die Geräte zur Messung von Vitalparametern klein, kompakt und bedienungsfreundlich gehalten werden müssen. Geräte, die zur Eingabe eine Benutzeroberfläche zur Eingabe von PIN-Codes oder dergleichen für das Bluetooth-Pairing aufweisen, scheiden als Geräte zur Messung von Vitalparametern in der Regel aus, da sie zu groß und störanfällig sind.

Die europäische Offenlegungsschrift EP 2 717 756 A2 offenbart einen Sensor zur Messung eines Vitalparameters im Gehörgang eines Menschen. Der Sensor hat jedoch nur eine Möglichkeit zur gesicherten Datenübertragung auf ein bluetoothfähiges Gerät, wenn vorher ein Pairing durchgeführt worden ist. Das Pairing ist hier jedoch die Angriffsstelle.

Die deutsche Offenlegungsschrift Nr. DE 10 2019 001 483 A1 offenbart eine automatisierte Methode zur Bluetoothpaarung zwischen einem Patientensensor und einem Patientenmonitor, bei der das Andocken des Sensors an die Ladevorrichtung sowohl vom Monitor als auch vom Sensor erfasst wird, um beide Geräte in einen Bereitschaftszustand zu versetzen und anschließend automatisch eine Bluetoothverbindung herzustellen..

Die US-Patentanmeldung Nr. US 2020/138288 A1 offenbart medizinische Überwachungssensoren, die aus einem wegwerfbaren Sensorbausatz und einem wiederverwendbaren Kopplungsgerät bestehen. Der wegwerfbare Sensor kann physiologische Daten des Patienten erfassen und gleichzeitig Energie für das wiederverwendbare Kopplungsgerät bereitstellen. Das wiederverwendbare Gerät kann eine drahtlose Verbindung zu einem Überwachungsgerät herstellen. Sobald das wiederverwendbare Kopplungsgerät die physiologischen Daten vom wegwerfbaren Sensor erhält, kann es diese Daten drahtlos an ein Computergerät übertragen.

### Darstellung der Erfindung, Aufgabe, Lösung, Vorteile

Ausgehend von den vorgenannten Überlegungen liegt der vorliegenden Erfindung deshalb die Aufgabe zugrunde, Übertragungseinheiten für ein Signal außerhalb der Bandbreite im Rahmen einer Bluetooth-Paarung von mindestens zwei bluetoothfähigen Geräten, ein lichtundurchlässiges Gerät zur Messung von Vitalparametern, ein System und ein Verfahren zur Bluetooth-Paarung bereitzustellen, bei denen auf die manuelle Eingabe von Authentifizierungscodes bzw. zur ungesicherten Übertragung verzichtet werden kann und so die oben geschilderten Problem vermieden werden können.

In einem ersten Aspekt betrifft die vorliegende Erfindung deshalb eine Übertragungseinheit für ein Signal außerhalb der Bandbreite im Rahmen einer Bluetooth-Paarung von mindestens zwei bluetoothfähigen Geräten wie in Anspruch 1 definiert.

In einer bevorzugten Implementierung kann das Signal auch zusätzlich oder gegebenenfalls alternativ zum dem Bluetooth-Paarungscode gerätespezifische oder patientenspezifische Daten repräsentieren, wie beispielsweise eine Konfiguration oder eine Patienten-Kennnummer (Patienten-ID).

Erfindungsgemäß wird unter einem Signal außerhalb der Bandbreite im Rahmen einer Bluetooth-Paarung von mindestens zwei bluetoothfähigen Geräten jedes Signal verstanden, dessen Frequenz sich außerhalb der im Rahmen einer Bluetooth-Paarung typischerweise verwendeten Frequenz-Bandbreite (out-of Band) befindet.

Erfindungsgemäß wird unter lichtundurchlässige Aufnahme jede lichtundurchlässige Aufnahme bzw. Ausnehmung verstanden, welche mechanisch oder durch andere Mittel gegenüber der Umgebung der Übertragungseinheit eine Abschirmung der Signalübertragung zwischen dem bluetoothfähigen Gerät und der Lichtquelle bzw. dem Lichtdetektor ermöglichen kann. Mit anderen Worten wird ein ungewollter Lichtsignalempfang durch dritte Geräte außerhalb des Systems Übertragungseinheit-bluetoothfähiges Gerät bei Positionierung des lichtundurchlässigen Gerätes in der lichtundurchlässigen Aufnahme verhindert.

Erfindungsgemäß wird unter Bluetooth-Paarungscode jeder Paarungscode für eine Bluetooth-Verbindung verstanden, welche eine gesicherte Verbindung zwischen zwei bluetoothfähigen Geräten bereitstellen kann. Beispielsweise kann der Bluetooth-Paarungscode ein vierstelliger oder sechsstelliger oder achtstelliger Paarungscode sein. Typerweise ist der Bluetooth-Paarungscode ein vierstelliger alphanumerischer Code.

Durch eine Out-of-Band-Verwaltung außerhalb des Bluetooth-Frequenzbereiches kann ein Betreiber eines Netzwerks beim Zugriff auf Netzwerkkomponente Vertrauensgrenzen festlegen. Die Out-of-Band-Verwaltung kann auch verwendet werden, um die Konnektivität (einschließlich der Möglichkeit, den Status einer Netzwerkkomponente zu bestimmen) unabhängig vom Status anderer In-Band-Netzwerkkomponenten sicherzustellen.

In einem zweiten Aspekt betrifft die vorliegende Erfindung ein Gerät zur Messung von Vitalparametern wie in Anspruch 2 definiert.

Erfindungsgemäß wird unter lichtundurchlässigem Gerät zur Messung von Vitalparametern, jedes Gerät verstanden, welches zur Messung von Vitalparametern an einem Menschen oder an einem Tier verwendet werden kann. Erfindungsgemäß kann als lichtundurchlässiges Gerät zur Messung von Vitalparametern gemäß dem zweiten Aspekt der Erfindung das bluetoothfähige Gerät gemäß dem ersten Aspekt der Erfindung verwendet werden.

Erfindungsgemäß wird unter Lichtdetektor jede Vorrichtung verwenden, welche ein informationstragendes Lichtsignal detektieren kann. In diesem Fall kann die Lichtsignalübertragung besonders zuverlässig und sicher erreicht werden.

Vorzugsweise ist das Lichtsignal ein Lichtsignal mit definierter Amplitude und Frequenz. Das Lichtsignal kann entweder ein monochromatisches Lichtsignal sein oder ein polychromatisches Lichtsignal. Das polychromatische Lichtsignal kann ein spezifisches, für das bluetoothfähige Gerät passendes LED-Farbspektrum aufweisen. Im Idealfall ist das Lichtsignal zeit- und wertdiskret. Beispielsweise kann das Lichtsignal ein Bluetooth-Paarungscode kodieren. Zusätzlich oder alternativ kann das Lichtsignal auch Parameter für die Konfiguration und/oder eine Patienten-ID kodieren.

Das lichtundurchlässige Gerät zur Messung von Vitalparametern kann so konfiguriert sein, dass der Lichtdetektor ein Lichtsignal empfangen kann, welches einen Bluetooth-Paarungscode und/oder gerätespezifische oder patientenspezifische Daten repräsentiert. Der Bluetooth-Paarungscode kann an ein weiteres Gerät ausgesendet werden. Vorteilhafterweise kann der mit dem Prozessor verbundene Lichtdetektor, beispielsweise eine Photodiode, sowohl zur Messung von Vitalparametern, als auch zum Empfangen eines Lichtsignals verwendet werden, der einen Bluetooth-Paarungscode repräsentiert.

In einer bevorzugten Implementierung weist das lichtundurchlässige Gerät zur Messung von Vitalparametern zusätzlich eine mit dem Prozessor verbundene Lichtquelle zum Aussenden eines Lichtsignals auf. Das Speichermittel kann vorzugsweise Instruktionen für den Prozessor umfassen, ein Lichtsignal auszusenden, welches einen Bluetooth-Paarungscode und/oder gerätespezifische oder patientenspezifische Daten repräsentiert.

Das lichtundurchlässige Gerät zur Messung von Vitalparametern kann auch so konfiguriert sein, dass eine Lichtquelle ein Lichtsignal aussenden kann, welches einen Bluetooth-Paarungscode und/oder gerätespezifische oder patientenspezifische Daten repräsentiert. Dieser Paarungscode kann dazu dienen, in einem weiteren Gerät, welches mit einem entsprechenden Signaldetektor ausgebildet ist, einen Bluetooth-Paarungscode bereitzustellen, welcher eine Repräsentation des ausgesendeten Lichtsignals darstellt. In diesem Fall kann das Gerät als Master konfiguriert sein.

Vorzugsweise sind die Speichermittel zur Speicherung mindestens eines Bluetooth-Paarungscodes und/oder gerätespezifische oder patientenspezifische Daten ausgebildet. **In** einer bevorzugten Implementierung können die Speichermittel zur Speicherung einer Vielzahl von Bluetooth-Paarungscodes und/oder gerätespezifischer oder patientenspezifischer Daten ausgebildet sein, welche mit zeit- und gerätespezifischen Informationen logisch verknüpft sein können.

Als Lichtdetektor kann jedes elektronische Bauelement verwendet werden, welches Licht unter Benutzung eines photoelektrischen Effekts in ein elektrisches Signal umwandeln kann oder einen von einer einfallenden Lichtstrahlung abhängigen elektrischen Widerstand zeigt. Erfindungsgemäß kann der Lichtdetektor aus der Gruppe ausgewählt sein, bestehend aus Photozellen, Photomultipliern, Mikrokanalplatten-Photomultipliern, CMOS-Sensoren, CCD-Sensoren, Photodioden, Fototransistoren und Fotowiderständen. Idealerweise ist der Lichtdetektor eine anorganische oder organische Photodiode oder ein Phototransistor.

Als Lichtquelle kann jedes elektronische Bauelement verwendet werden, welches Licht mit einer definierten Wellenlängenverteilung nach Frequenz und Intensität aussenden kann. Erfindungsgemäß ist die Lichtquelle vorzugsweise eine Leuchtdiode, d.h. eine lichtemittierende Diode.

Vorzugsweise ist das lichtundurchlässige Gerät zur Messung von Vitalparametern im Gehörgang ausgebildet.

In einem dritten Aspekt betrifft die Erfindung ein System wie in Anspruch 7 definiert.

Vorzugsweise kann die Abschirmung der Signalübertragung dadurch erreicht werden, dass das lichtundurchlässige Gerät und die lichtundurchlässige Aufnahme der Übertragungseinheit beim Positionieren des lichtundurchlässigen Gerätes in der lichtundurchlässigen Aufnahme der Übertragungseinheit der Lichtempfänger und der Lichtdetektor im Inneren des Systems in räumlicher Nähe positioniert sind. In diesem Fall können die Übertragungseinheit und das lichtundurchlässige Gerät selbst als Abschirmung dienen. Vorzugsweise kann das lichtundurchlässige Gerät formschlüssig in die lichtundurchlässige Aufnahme der Übertragungseinheit positioniert sein, so dass sich eine sehr gute Abschirmung ergibt. Alternativ kann die lichtundurchlässige Aufnahme der Übertragungseinheit auch eine konkave, lichtdurchlässige Aussparung oder eine tubulare, lichtundurchlässige Aussparung sein, in die ein konvexes oder elongiertes Bluetooth-Modul eingelegt oder eingeschoben werden kann. In diesem Fall stellen die lichtundurchlässige Aussparung und der räumliche Abschluss des eingelegten bzw. eingeschobenen Bluetooth-Moduls die Abschirmung bereit.

Vorzugsweise weist die Übertragungseinheit und/oder das lichtundurchlässige Gerät zusätzlich mechanische oder magnetische Positionierungs- oder Arretierungshilfen auf. Mithilfe der Positionierungs- oder Arretierungshilfen kann der Lichtempfänger und der Lichtdetektor in räumlicher Nähe positioniert bzw. arretiert, so dass sich eine möglichst effiziente Übertragung ergibt. Beispielsweise kann das lichtundurchlässige Gerät mit Magneten als Positionierungshilfen lösbar mit der Übertragungseinheit verbunden werden, wobei der Lichtempfänger und der Lichtdetektor auf abgeschirmte Weise in räumliche Nähe gebracht werden können. Alternativ kann das lichtundurchlässige Gerät mit mechanischen Haltemitteln, z.B. Klettverbindungen oder durch Kraftschluss, als Positionierungshilfen lösbar mit der Übertragungseinheit verbunden werden, wobei der Lichtempfänger und der Lichtdetektor auf abgeschirmte Weise in räumliche Nähe gebracht werden können.

Das System kann somit neben dem eigentlichen lichtundurchlässige Gerät zur Messung von Vitalparametern eine Übertragungseinheit umfassen, die mit einer lichtundurchlässigen Aufnahme ausgebildet ist. In dieser lichtundurchlässigen Aufnahme können sich eine Lichtquelle oder ein Lichtdetektor oder eine Kombination von beidem befinden, sodass ein in der lichtundurchlässigen Aufnahme positioniertes lichtundurchlässige Gerät sowohl einen Bluetooth-Paarungscode über ein entsprechendes Signal empfangen kann (Slave) als auch einen Bluetooth-Paarungscode über ein entsprechendes Signal generieren kann (Master).

Die Übertragungseinheit kann in ein weiteres, bluetoothfähiges Gerät integriert sein, bevorzugt liegt sie als separate Übertragungseinheit vor.

Vorzugsweise ist die Übertragungseinheit dazu ausgebildet, ein definiertes Lichtsignal auszusenden, um in dem lichtundurchlässigen Gerät einen Bluetooth-Paarungscode bereitzustellen.

Vorzugsweise ist die Übertragungseinheit dazu ausgebildet, ein definiertes Lichtsignal zu empfangen, um einen Bluetooth-Paarungscode bereitzustellen.

**In** einem vierten Aspekt betrifft die vorliegende Erfindung ein Verfahren zum Verbinden des erfindungsgemäßen lichtundurchlässigen Geräts zur Messung von Vitalparametern mit einem weiteren bluetoothfähigen Gerät wie in Anspruch 9 definiert.

Vorzugsweise weist das Verfahren zum Verbinden eines erfindungsgemäßen lichtundurchlässigen Geräts zur Messung von Vitalparametern mit einem weiteren bluetoothfähigen Gerät zusätzlich den folgenden Schritt a') auf.

Der Schritt a') beinhaltet das Aussenden eines definierten Signals durch das lichtundurchlässige Gerät zur Messung von Vitalparametern, wobei das definierte Lichtsignal von einer Lichtquelle des lichtundurchlässigen Geräts an einen Lichtdetektor übermittelt wird, wobei die Lichtquelle und der Lichtdetektor abgeschirmt sind.

Vorzugsweise verwendet das Verfahren gemäß dem vierten Aspekt der vorliegenden Erfindung das System gemäß dem dritten Aspekt der vorliegenden Erfindung.

### Bevorzugte Ausführung der Erfindung

Im Folgenden werden beispielhaft und nicht abschließend einige besondere Ausführungsformen der Erfindung beschrieben. Die besonderen Ausführungsformen dienen nur zur Erläuterung des allgemeinen erfinderischen Gedankens, jedoch beschränken sie die Erfindung nicht.

In einer möglichen Ausführungsform weist die Übertragungseinheit eine Aufnahme für ein bluetoothfähiges Gerät auf, wobei die Aufnahme mit einer LED-Lichtquelle ausgebildet ist. Ein Gerät zur Messung von Vitalparametern in einer möglichen Ausführung weist ein Bluetooth-Modul zum Paaren des Geräts mit einem weiteren, bluetoothfähigen Gerät zur drahtlosen Datenübertragung auf, einen mit dem Bluetooth-Modul verbundenen Prozessor, eine mit dem Prozessor verbundenen anorganische Photodiode als Signaldetektor und mit dem Prozessor verbundene Speichermittel, welche Instruktionen für den Prozessor umfassen. Die Instruktionen dienen dazu, einen Bluetooth-Paarungscode und/oder gerätespezifische oder patientenspezifische Daten zu empfangen, welcher ein empfangenes Signal repräsentiert. Die Speichermittel enthalten auch Instruktionen für den Prozessor umfassen, ein Signal auszusenden, welches einen Bluetooth-Paarungscode und/oder gerätespezifische oder patientenspezifische Daten repräsentiert. Die Speichermittel können den empfangenen bzw. zu sendenden Bluetooth-Paarungscode und/oder gerätespezifische oder patientenspezifische Daten speichern. Das Gerät zur Messung von Vitalparametern ist der Form und der Größe nach angepasst, um Vitalparameter im Gehörgang messen zu können.

Zur Übertragung des Bluetooth-Paarungscodes und/oder gerätespezifischer oder patientenspezifischer Daten kann das Gerät mit dem Bluetooth-Modul in die abgeschirmte Aufnahme der Übertragungseinheit eingelegt oder eingesteckt werden. Eine sichere, unabfangbare Übertragung des Bluetooth-Paarungscodes und/oder gerätespezifischer oder patientenspezifischer Daten ausgehend von der LED-Lichtquelle zur anorganischen Photodiode ist hierdurch möglich. Fremde Dritte haben bei dem Übertragungsvorgang keine Möglichkeit, das Signal optisch oder auf andere Weise abzufangen. Das Signal kann durch Änderung der Lichtintensität, der Übertragungsfrequenz oder einer Kombination davon übertragen werden. Die Photodiode erkennt als Sensor den Bluetooth-Paarungscode und nutzt diese für alle späteren Bluetooth-Verbindungen als Verschlüsselungscode.

Die Kombination aus dem Übertragungseinheit und dem Gerät zur Messung von Vitalparametern können in einem System verwendet werden, um abgeschirmt Signale empfangen und/oder aussenden kann, welche einen Bluetooth-Paarungscode und/oder gerätespezifische oder patientenspezifische Daten repräsentieren, oder können auch in einem Verfahren verwendet werden, in welchem das Gerät mit einem Mobiltelefon als einem weiteren bluetoothfähigen Gerät verbunden wird.

## Patentansprüche

1. Übertragungseinheit für ein Signal außerhalb der Bandbreite im Rahmen einer Bluetooth-Paarung von mindestens zwei bluetoothfähigen Geräten, wobei die Übertragungseinheit mit mindestens einer lichtundurchlässigen Aufnahme für ein bluetoothfähiges Gerät ausgebildet ist, wobei die lichtundurchlässige Aufnahme mit einer Lichtquelle und/oder mit einem Lichtdetektor ausgebildet ist, derart, dass ein in der lichtundurchlässigen Aufnahme positioniertes bluetoothfähiges Gerät abgeschirmt Lichtsignale empfangen und/oder aussenden kann, welche einen Bluetooth-Paarungscode und/oder gerätespezifische oder patientenspezifische Daten, wie beispielsweise eine Konfiguration oder eine Patienten-Kennnummer, repräsentieren.

2. Lichtundurchlässiges Gerät zur Messung von Vitalparametern, umfassend:
ein Bluetooth-Modul zum Paaren des lichtundurchlässigen Geräts mit einem weiteren, bluetoothfähigen Gerät zur drahtlosen Datenübertragung,
einen mit dem Bluetooth-Modul verbundenen Prozessor;
einen mit dem Prozessor verbundenen Lichtdetektor; und
mit dem Prozessor verbundene Speichermittel, welche Instruktionen für den Prozessor umfassen, einen Bluetooth-Paarungscode und/oder
gerätespezifische oder patientenspezifische Daten zu empfangen, welcher ein empfangenes Lichtsignal repräsentiert.

3. Lichtundurchlässiges Gerät gemäß Anspruch 2, zusätzlich umfassend eine mit dem Prozessor verbundene Lichtquelle zum Aussenden eines Lichtsignals, wobei die Speichermittel Instruktionen für den Prozessor umfassen, ein Lichtsignal auszusenden, welches einen Bluetooth-Paarungscode und/oder gerätespezifische oder patientenspezifische Daten repräsentiert.

4. Lichtundurchlässiges Gerät gemäß einem der Ansprüche 2 oder 3, wobei die Speichermittel zur Speicherung mindestens eines Bluetooth-Paarungscodes und/oder gerätespezifische oder patientenspezifische Daten ausgebildet sind.

5. Lichtundurchlässiges Gerät gemäß einem der vorhergehenden Ansprüche, wobei der Lichtdetektor als eine anorganische oder organische Photodiode oder als ein Phototransistor ausgebildet ist und/oder die Lichtquelle als Leuchtdiode ausgebildet ist.

6. Lichtundurchlässiges Gerät gemäß einem der vorhergehenden Ansprüche, wobei das lichtundurchlässige Gerät zur Messung von Vitalparametern im Gehörgang ausgebildet ist.

7. System, umfassend
ein lichtundurchlässiges Gerät zur Messung von Vitalparametern gemäß einem der Ansprüche 2 bis 6, und
eine Übertragungseinheit gemäß Anspruch 1, wobei die lichtundurchlässige Aufnahme mit einer Lichtquelle und/oder mit einem Lichtdetektor ausgebildet ist, derart dass ein in der lichtundurchlässigen Aufnahme positioniertes lichtundurchlässiges Gerät zur Messung von Vitalparametern abgeschirmt Lichtsignale empfangen und/oder aussenden kann, welche einen Bluetooth-Paarungscode und/oder gerätespezifische oder patientenspezifische Daten repräsentieren.

8. System gemäß Anspruch 7, wobei die Übertragungseinheit dazu ausgebildet ist, ein definiertes Lichtsignal auszusenden, um in dem lichtundurchlässigen Gerät einen Bluetooth-Paarungscode und/oder gerätespezifische oder patientenspezifische Daten bereitzustellen und/oder dazu ausgebildet ist, ein definiertes Lichtsignal zu empfangen, um einen Bluetooth-Paarungscode und/oder gerätespezifische oder patientenspezifische Daten bereitzustellen.

9. Verfahren zum Verbinden eines lichtundurchlässigen Geräts zur Messung von Vitalparametern gemäß einem der Ansprüche 2 bis 6 mit einem weiteren bluetoothfähigen Gerät, umfassend:
a) Empfangen eines definierten Lichtsignals durch das lichtundurchlässige Gerät zur Messung von Vitalparametern, wobei das definierte Lichtsignal von einer Lichtquelle an den Lichtdetektor des lichtundurchlässigen Geräts zur Messung von Vitalparametern übermittelt wird, wobei die Lichtquelle und der Lichtdetektor abgeschirmt sind,
b) Auswerten des definierten Lichtsignals als ein Bluetooth-Paarungscode, und
c) Verwenden des Bluetooth-Paarungscodes, um zwischen dem Gerät zur Messung von Vitalparametern und dem weiteren bluetoothfähigen Gerät eine Bluetooth-Verbindung herzustellen.

10. Verfahren gemäß Anspruch 9 zusätzlich umfassend den Schritt:
a') Aussenden eines definierten Lichtsignals durch das Gerät zur Messung von Vitalparametern, wobei das definierte Lichtsignal von einer Lichtquelle des lichtundurchlässigen Geräts an einen Lichtdetektor übermittelt wird, wobei die Lichtquelle und der Lichtdetektor abgeschirmt sind.

11. Verfahren gemäß einem der Ansprüche 9 oder 10, unter Verwendung des Systems gemäß einem der Ansprüche 7 oder 8.

## Claims

1. Transmission unit for a signal outside the bandwidth within the framework of a Bluetooth pairing of at least two Bluetooth-capable devices, wherein the transmission unit is formed with at least one light-impermeable receptacle for a Bluetooth-capable device, wherein the light-impermeable receptacle is formed with a light source and/or with a light detector, in such a way that a Bluetooth-capable device positioned in the light-impermeable receptacle can receive and/or emit light signals in a shielded manner, which light signals represent a Bluetooth pairing code and/or device-specific or patient-specific data, such as for example a configuration or a patient identification number.

2. Light-impermeable device for measuring vital parameters, comprising:
a Bluetooth module for pairing the light-impermeable device with a further, Bluetooth-capable device for wireless data transmission,
a processor connected to the Bluetooth module;
a light detector connected to the processor; and
memory means connected to the processor, which comprise instructions for the processor to receive a Bluetooth pairing code and/or device-specific or patient-specific data, which represents a received light signal.

3. Light-impermeable device according to claim 2, additionally comprising a light source connected to the processor for emitting a light signal, wherein the memory means comprise instructions for the processor to emit a light signal which represents a Bluetooth pairing code and/or device-specific or patient-specific data.

4. Light-impermeable device according to either of claims 2 or 3, wherein the memory means are designed for storing at least one Bluetooth pairing code and/or device-specific or patient-specific data.

5. Light-impermeable device according to any one of the preceding claims,
wherein the light detector is designed as an inorganic or organic photodiode or as a phototransistor and/or the light source is designed as a light-emitting diode.

6. Light-impermeable device according to any one of the preceding claims,
wherein the light-impermeable device is designed for measuring vital parameters in the auditory canal.

7. System, comprising
a light-impermeable device for measuring vital parameters according to any one of claims 2 to 6, and
a transmission unit according to claim 1, wherein the light-impermeable receptacle is formed with a light source and/or with a light detector, in such a way that a light-impermeable device for measuring vital parameters positioned in the light-impermeable receptacle can receive and/or emit light signals in a shielded manner, which light signals represent a Bluetooth pairing code and/or device-specific or patient-specific data.

8. System according to claim 7, wherein the transmission unit is designed to emit a defined light signal in order to provide a Bluetooth pairing code and/or device-specific or patient-specific data in the light-impermeable device and/or is designed to receive a defined light signal in order to provide a Bluetooth pairing code and/or device-specific or patient-specific data.

9. Method for connecting a light-impermeable device for measuring vital parameters according to any one of claims 2 to 6 to a further Bluetooth-capable device, comprising:
a) receiving a defined light signal by the light-impermeable device for measuring vital parameters, wherein the defined light signal is transmitted from a light source to the light detector of the light-impermeable device for measuring vital parameters, wherein the light source and the light detector are shielded,
b) evaluating the defined light signal as a Bluetooth pairing code, and
c) using the Bluetooth pairing code in order to establish a Bluetooth connection between the device for measuring vital parameters and the further Bluetooth-capable device.

10. Method according to claim 9, additionally comprising the step:
a') emitting a defined light signal by the device for measuring vital parameters, wherein the defined light signal is transmitted from a light source of the light-impermeable device to a light detector, wherein the light source and the light detector are shielded.

11. Method according to either of claims 9 or 10, using the system according to either of claims 7 or 8.

## Revendications

1. Unité de transmission pour un signal en dehors de la largeur de bande dans le cadre d'un appairage Bluetooth d'au moins deux dispositifs compatibles Bluetooth, l'unité de transmission étant réalisée avec au moins un réceptacle opaque à la lumière pour un dispositif compatible Bluetooth, le réceptacle opaque à la lumière étant réalisé avec une source de lumière et/ou avec un détecteur de lumière, de telle sorte qu'un dispositif compatible Bluetooth positionné dans le réceptacle opaque à la lumière puisse recevoir et/ou émettre de manière protégée des signaux lumineux qui représentent un code d'appairage Bluetooth et/ou des données spécifiques au dispositif ou spécifiques au patient, telles que par exemple une configuration ou un numéro d'identification de patient.

2. Dispositif opaque à la lumière pour la mesure de paramètres vitaux, comprenant :
un module Bluetooth pour appairer le dispositif opaque à la lumière avec un autre dispositif compatible Bluetooth en vue d'une transmission de données sans fil,
un processeur relié au module Bluetooth ;
un détecteur de lumière relié au processeur ; et
des moyens de mémoire reliés au processeur, qui comprennent des instructions destinées au processeur pour recevoir un code d'appairage Bluetooth et/ou des données spécifiques au dispositif ou spécifiques au patient, qui représente un signal lumineux reçu.

3. Dispositif opaque à la lumière selon la revendication 2, comprenant en outre une source de lumière reliée au processeur pour émettre un signal lumineux, les moyens de mémoire comprenant des instructions destinées au processeur pour émettre un signal lumineux qui représente un code d'appairage Bluetooth et/ou des données spécifiques au dispositif ou spécifiques au patient.

4. Dispositif opaque à la lumière selon l'une des revendications 2 ou 3, les moyens de mémoire étant réalisés pour mémoriser au moins un code d'appairage Bluetooth et/ou des données spécifiques au dispositif ou spécifiques au patient.

5. Dispositif opaque à la lumière selon l'une quelconque des revendications précédentes, le détecteur de lumière étant réalisé sous la forme d'une photodiode inorganique ou organique ou d'un phototransistor et/ou la source de lumière étant réalisée sous la forme d'une diode électroluminescente.

6. Dispositif opaque à la lumière selon l'une quelconque des revendications précédentes, le dispositif opaque à la lumière étant réalisé pour la mesure de paramètres vitaux dans le conduit auditif.

7. Système, comprenant
un dispositif opaque à la lumière pour la mesure de paramètres vitaux selon l'une quelconque des revendications 2 à 6, et
une unité de transmission selon la revendication 1, le réceptacle opaque à la lumière étant réalisé avec une source de lumière et/ou avec un détecteur de lumière, de telle sorte qu'un dispositif opaque à la lumière pour la mesure de paramètres vitaux positionné dans le réceptacle opaque à la lumière puisse recevoir et/ou émettre de manière protégée des signaux lumineux qui représentent un code d'appairage Bluetooth et/ou des données spécifiques au dispositif ou spécifiques au patient.

8. Système selon la revendication 7, l'unité de transmission étant réalisée pour émettre un signal lumineux défini afin de fournir dans le dispositif opaque à la lumière un code d'appairage Bluetooth et/ou des données spécifiques au dispositif ou spécifiques au patient et/ou étant réalisée pour recevoir un signal lumineux défini afin de fournir un code d'appairage Bluetooth et/ou des données spécifiques au dispositif ou spécifiques au patient.

9. Procédé pour connecter un dispositif opaque à la lumière pour la mesure de paramètres vitaux selon l'une quelconque des revendications 2 à 6 à un autre dispositif compatible Bluetooth, comprenant :
a) la réception d'un signal lumineux défini par le dispositif opaque à la lumière pour la mesure de paramètres vitaux, le signal lumineux défini étant transmis d'une source de lumière au détecteur de lumière du dispositif opaque à la lumière pour la mesure de paramètres vitaux, la source de lumière et le détecteur de lumière étant protégés,
b) l'évaluation du signal lumineux défini en tant que code d'appairage Bluetooth, et
c) l'utilisation du code d'appairage Bluetooth afin d'établir une connexion Bluetooth entre le dispositif pour la mesure de paramètres vitaux et l'autre dispositif compatible Bluetooth.

10. Procédé selon la revendication 9, comprenant en outre l'étape :
a') l'émission d'un signal lumineux défini par le dispositif pour la mesure de paramètres vitaux, le signal lumineux défini étant transmis d'une source de lumière du dispositif opaque à la lumière à un détecteur de lumière, la source de lumière et le détecteur de lumière étant protégés.

11. Procédé selon l'une des revendications 9 ou 10, utilisant le système selon l'une des revendications 7 ou 8.
